Europäisches Patentamt

European Patent Office

Office européen des brevets

(19)

(11) Publication number: **0 181 524**
**A2**

(12) # EUROPEAN PATENT APPLICATION

(21) Application number: 85113254.8

(22) Date of filing: 18.10.85

(51) Int. Cl.⁴: **C 12 P 21/02**

(30) Priority: 19.10.84 JP 218643/84

(43) Date of publication of application:
21.05.86 Bulletin 86/21

(84) Designated Contracting States:
AT BE CH DE FR GB IT LI NL SE

(71) Applicant: Eisai Co., Ltd.
6-10, Koishikawa 4-chome Bunkyo-ku
Tokyo 112(JP)

(72) Inventor: Ohsawa, Toshiaki
9-13, Akatsuka-shinmachi 1-chome
Itabashi-ku Tokyo(JP)

(72) Inventor: Kobayashi, Yoshiro
3-7-304, Gyodacho 2-chome
Funabashi-shi Chiba(JP)

(72) Inventor: Asada, Makoto
7-32, Oshitatemachi 5-chome
Fuchu-shi Tokyo(JP)

(74) Representative: Eitle, Werner, Dipl.-Ing. et al,
Hoffmann, Eitle & Partner Patentanwälte Arabellastrasse
4
D-8000 München 81(DE)

(54) Novel lymphokine.

(57) A lymphokine has been produced from human T cell hybridoma derived from human T cells treated with keyhole limpet hemocyanin and has the following physicochemical properties:

(i) it has a molecular weight of approximately 15,000 and 30,000,

(ii) it is chemically unstable under an acidic condition,

(iii) it exerts a natural killer cell activating activity,

(iv) it exhibits no interleukin 2 activity, and

(v) it exhibits no interferon activity.

Novel Lymphokine


This invention relates to a novel lymphokine produced by human T cells or human T cell hybridoma.

"Lymphokine" is a generic name for nonantibody soluble substances which are liberated by lymphocytes activated by an antigen or a mitogen and have various biological activities. In a narrow sense, lymphokine represents a group of factors which are produced by activated T cells and act as a primary medium in cellular immunity. However lymphokine has been recently considered to further represent another group of factors relating to intercellular reactions in immune responses. The former group includes, e.g., macrophage migration inhibition factor (MIF), macrophage activating factor (MAF) and lymphotoxin (LT) while the latter group includes, e.g., repressible factors for antibody production, helper factor and T cell growth factor (IL-2).

- 2 -

Lymphokines are obtained from a supernatant of a culturing broth of normal lymphocytes in a highly limited amount and at a highly limited purity so that the roles of many lymphokines are yet not completely known. However many workers have attempted to detect T tumor strains capable of producing lymphokines and to establish T cell hybridomas since some lymphokines may be clinically available when produced in a large amount. As a result, a T tumor strain (Jurkat-FHCRC) capable of producing IL-2 has been found. Further monoclonal antiIL-2 antibody has been prepared and applied to the growth of T cells. Furthermore the role of the IL-2 in inducing the differentiation of allo-killer T cells has been established and mRNA and cDNA have been isolated and identified. These works have been established in line with studies on interferon. It is expected that a lymphokine obtained in a large amount and in a pure state with the help of genetic engineering as described above would not only be available in the clinical field but serve as a very powerful tool in the elucidation of the relation between its structure and function, the mechanism of its function and its role in vivo.

It is known that natural killer cells would exert a destructive effect on certain cancerous cells and much attention is denoted to lymphokines affecting said cells. For example, IL-2 and interferon have been reported to induce an increase in the activity of natural killer cells. It has been proposed that the natural killer cells might produce some factors toxic to the cancerous cells thus exerting the above-

0181524

- 4 -

mentioned anticancerous effect. It has been further proposed that said factors might be lymphotoxin in a certain form. On the other hand, a factor which is liberated when human lymphocytes are contacted with cancerous cells and strengthens the cytotoxicity of lymphocytes is considered to be lymphotoxin. Furthermore it has been reported that lymphotoxin would enhance the sensitivity of cancerous cells against natural killer cells. These facts suggest that lymphotoxin might play an important role both directly and indirectly as a lymphokine which affects the activity of natural killer cells.

To further illustrate these findings on natural killer cells and lymphotoxin, the following references 1) to 8) will be given.

1) Herberman, R.B., Djeu, J.K., Kay, H.D., Ortaldo, J.R., Riccardi, C., Bonnard, G.D. Holden, H.T., Fagnani, R., Santoni, A. and Puccetti, P., Immunol. Rev., 44, 43 (1979).

2) Vose, B.M. Riccardi, C., Bonnard, G.D. and Herberman, R.B., J. Immunol., 130, 768 (1983).

3) Domzig, W., Stadler, B.M. and Herberman, R.B., J. Immunol., 130, 1970 (1983).

4) Wright, S.C., Weitzeu, M.L., Kahle, R., Granger, G.A. and Bonavida, B., J. Immunol., 130, 2479 (1983).

5) Kedar, E., Ikejiri, B.L., Sredni, B., Bonavida, B. and Herberman, R.B., Cell. Immuno., 69, 305 (1982)

6) Weitzen, M.L. Yamamoto, R.S. and Granger, G.A., Cell. Immuno., 77, 30 (1983)

7) Peter, H.H., Eife, R.F. and Kaldeu, J.R., J. Immunol., 116, 342 (1976); and

8) Ransom, J.H. and Evans, C.H., Int. J. Cancer, 29, 451 (1982).

Under these circumstances, we have attempted various studies on the assumption that there may be a lymphokine which can activate natural killer cells. We have previously established several cell lines relating to lymphokine-producing human T cell hybridoma and disclosed the presence of lymphokines such as MIF and MAF (cf. references 9) to 11) as will be cited hereinbelow). At the same time, we have tried to prepare human T cell hybridoma capable of producing a natural killer cell activating factor by a special treatment different from those used in the previous studies. Consequently we have found that human T cell hybridoma derived from human T cells treated with keyhole limpet hemocyanin would produce a natural killer cell activating factor, thus completing the present invention.

Accordingly it is an object of the present invention to provide a novel natural killer cell activating factor. To achieve the above object, the present invention discloses a lymphokine which is produced by human T cell hybridoma derived from human T cells treated with keyhole limpet hemocyanin and has the following physicochemical properties:

(i) it has a molecular weight of approximately 15,000 and 30,000,

(ii) it is chemically unstable under an acidic condition,

(iii) it exerts a natural killer cell activating activity,

(iv) it exhibits no interleukin 2 activity, and

(v) it exhibits no interferon activity.

The following references 9) to 11) relating to lymphokine-producing human T cell hybridoma established by us by emetine-actinomycin D method are cited as reference to the present invention.

9) Kobayashi, Y., Asada, M., Higuchi, M. and Osawa, T., J. Immunol., 128, 2714 (1982).

10) Asada, M., Higuchi, M. Kobayashi, Y. and Osawa, T., Cell. Immunol., 77, 150 (1983) and

11) Higuchi, M., Asada, M., Kobayashi, Y. and Osawa, T., Cell. Immunol., 78, 257 (1983)

Constitution of the Invention:

Now the present invention will be described in detail.

The starting human T cells to use in the invention are easily available to a person skilled in the art.

The human T cells used in the present invention are lymphocytes of human peripheral blood or those isolated from human spleen or thymus. Although peripheral blood lymphocytes may be usually employed, only those in the form of mononuclear cells can be used, and further those capable of forming rosettes (i.e. rosette forming cells) and those adhering to plastics (i.e. adherent cells) are selected for use.

The present invention is characterized in that the human T cells are treated with keyhole limpet hemocyanin. The keyhole limpet hemocyanin (KLH), which is one of mytogens, is commercially available from Calbiochem-Behring Co., CA. The treatment of the human T cells with KLH comprises, e.g., sensitizing a mixture of the rosette forming cells and the adherent cells as described above by contacting with KLH in an appropriate medium. After the sensitization, these cells may further grow in the medium containing IL-2.

The human T cell hybridoma relating to the present invention may be prepared by fusing the human T cells treated with KLH by an appropriate method, such as

- 8 -

0181524

HAT selection method or emetine-actinomycin D method, and screening the fused cells thus obtained according to the function of activating natural killer cells. The emetine-actinomycin D method developed by us may be performed as follows.  The human T cells treated with KLH are mixed with a human acute leukemia cell strain such as CEM in a ratio of 1 : 1 to 10 : 1. Then polyethylene glycol 4000 is added thereto to thereby fuse the cells followed by screening.  Cloning may be carried out by limiting dilution method. As will be described in the examples hereinbelow, we have obtained a strain named KC8-1 as the human .T cell hybrodima relating to the present invention. Further cloning of this KC8-1 according to the function of activating natural killer cells has resulted in two strains named KC8-1-9 and KC8-1-10. The applicant asked for deposition of strains (KC8-1, KC8-1-9 and KC8-1-10 with the Fermentation Research Institute.*

The lymphokine of the present invention, which occurs in the supernatant of a culturing broth of the human T cell hybridoma relating to the present invention, can be purified by concentrating the supernatant to a degree of a saturation of 90 % with ammonium sulfate, eluting the concentrate through a Blue Sepharose column (mfd. by Pharmacia AB) with PBS

*But the authority refused the proposed deposition because the strains fall outside the scope of micro-organisms that FERM can accept.

and collecting fractions capable of activating natural killer cells.

The molecular weight of the lymphokine thus purified was determined to be approximately 15,000 and 30,000 by eluting the lymphokine through a TSK G3000-SW column with PBS followed by gel filtration. It has been turned out that the lymphokine exhibits an activity of activating natural killer cells when determined in the process as will be described hereinbelow. Thus we have named the lymphokine of the present invention natural killer cell activating factor (NKAF). It has been further found that this activity would significantly fall when the lymphokine of the present invention is allowed to stand at a pH value of 2 for 24 hours, which suggests that the lymphokine of the present invention is chemically unstable under an acidic condition.

It has been further found that the lymphokine of the present invention exhibits neither IL-2 activity nor interferon activity both determined by the processes as will be described hereinbelow.

The lymphokine of the present invention seems to attack natural killer cells. That is, the lymphokine of the present invention could not exhibit any activity of activating natural killer cells in the process for determining said activity as will be described hereinbelow wherein plastic nonadherent PBS is treated with antiLeu-11 monoclonal antibody and rabbit complement to thereby previously remove natural killer cells Leu-11 surface antigen. This fact suggests that the lymphokine of the present invention requires natural cells as a target. The Leu-11 surface antigen of natural killer cells is described in the following references.

12) Phillips, J.H. and Babcock, G.F., Immunol. Letters, 6, 143 (1983).

13) Lanier, L.L., Le, A.M., Phillips, J.H., Warner, N.L. and Babcock, G.F., J. Immunol., 131, 1789 (1983).

14) Phillips, J.H., Le, A.M. and Lanier, L.L., J. Exp. Med., 159, 993 (1984).

Process for determining natural killer cell activating activity

Human peripheral blood lymphocytes nonadherent to plastics (referred to as plastics nonadherent PBL) are introduced into an RPMI-1640 medium containing 10 % of bovine fetal serum. A culturing supernatant of human T cell hybridoma is added thereto. The mixture thus obtained is adjusted to a total volume of 100 µl for use as a sample. The density of the plastics nonadherent PBL is adjusted to $5 \times 10^5$ cells/ml.

After culturing for 20 hours, 100 μl of a solution of K562 cells ($10^5$/ml) labeled with $^{51}$Cr is added to the culturing broth and the mixture is cultured for additional four hours. Then 100 μl of the culturing mixture is collected to determine free $^{51}$Cr therein. Separately a control experiment is performed in the following manner. 100 μl of an RPMI-1640 medium containing 10 % of bovine fetal serum is cultured for 20 hours and 100 μl of a solution of K562 cells ($10^5$/ml) labelled with $^{51}$Cr is added thereto. The mixture is cultured for additional four hours. Then 100 μl of the mixture is collected to determine free $^{51}$Cr therein.

The natural killer cell activity (hereinafter referred to as NK activity) is determined by substituting each value thus determined in the following equation:

NK activity = (b-a)/(c-a) x 100 % ;

wherein b represents the amount of free $^{51}$Cr (cpm) determined in the test sample, a represent the amount of free $^{51}$Cr (cpm) determined in the control sample and c represents the total amount of $^{51}$Cr present in 100 μl of the solution of K562 cells ($10^5$/ml) labeled with $^{51}$Cr. Each value is an average of three runs.

The procedure as described above is followed except that no human T cell hybridoma is added to determine the NK activity. Each value is an average of three runs. The NK activity in the case where the culturing supernatant of human T cell hybridoma is added is referred to as S while that of the case where no culturing supernatant of human T cell hybridoma is added is referred to as R. Thus the natural killer cell activating factor activity (hereinafter referred to as NKAF activity) according to the present invention is determined as follows:

NKAF activity = (S/R - 1) x 100 %.

In the present invention, an NKAF activity represents not a value as determined according to the above equation but a ratio of NK activities (i.e. R and S) unless otherwise stated.

Process for determining IL-2 activity

This activity is determined as a capability of a culturing broth of human T cell hybridoma to sustain the growth of mouse IL-2 dependent cells such as Lc7. That is, $5 \times 10^3$ Lc7 cells are introduced into 50 µl of an RPMI-1640 medium containing 10 % of bovine fetal serum and 50 µl of a sequentially diluted culturing broth of human T cell hybridoma is added thereto. The mixture is cultured for 20 hours and pulsed with 0.5 µCi of $^3$H-thymidine (specific activity of 0.5 Ci/mM, mfd by Amersham. England) to thereby determine incorporated radioactivity with a liquid scintillation counter.

The procedure as described above is followed except that no culturing supernatant of human T cell hybridoma is added, to thereby determine the radioactivity. In the present invention, an IL-2 activity represents a ratio of these two activities thus determined.

Process for determining interferon activity

This activity is practically determined as an antiviral activity against stomatitis virus. .That is, it is expressed as an decrease in the plaques formed on human amniotic cells by said virus. The procedure for the determination thereof is described in detail in the following references 15) and 16).

15) Hooks, J.J., Hayes, B.F., Petrich-Hooks, B., Dieh, L. F.,Gerrard, T.L. and Fcuuci, A.S., Blood, 59, 198 (1982).

**0181524**

16) Hooks, J.J., Moutsopoulos, M.; Geis, S., Stahl, N.I.; Decker J.L., Notkins, A.L., Immune interferon in the circulation of patients with autoimmune diseases, N. Engl., J. Med., 301, 5 (1979).

Brief Description of the Drawings:

Fig. 1 is a graph which shows the relationship between fraction No. in gel filtration and the natural killer cell activating factor activity (% increase) (see Example 1 - (h)).

Fig. 2 is a graph which shows the radioactivity and the natural killer cell activity of fractions eluted with various eluents (see Example 1 - (h)).

To further illustrate the present invention, the following examples will be given.

Example 1

(a) Preparation of human T cells

Human peripheral blood collected from a donor YK was laminated on a Ficoll-Urografin solution and mononuclear cells were collected therefrom by concentration gradient centrifugation by reference to the following literature.

17) Kawaguchi, T., Matsumoto, I. and Osawa, T., J. Biol. Chem., 249, 2786 (1974).

The sheep erythrocytes treated with 2-aminoethyl-isothiouronium hydrobromide (AET) were added to the mononuclear cells to form rosettes, thus isolating the rosette-forming cells by reference to the following literature.

18) Pellegrino, M.A., Ferrone, S., Dierich, M.P. and Reisfeld, R.A., Clin. Immunol. Immunophatol., 3, 324 (1975).

The above mononuclear cells were cultured in a plastics petri dish (3003; mfd. by Falcon Plastics, Bacton Dickinson Co., Cockenysvile, MD) for one hour. Then cells adhering to the surface of the plastics dish were collected and treated by adding mitomycin C (50 µg/ml) for 30 min to thereby prepare plastics adherent cells.

(b) KLH treatment

$10^6$/ml of the rosette forming cells and $10^5$/ml

of the plastics adherent cells each obtained in (a) were introduced into an RPNI-1640 medium containing 10 % of bovine fetal serum, 2mM of glutamine and 5 x $10^{-5}$ M of 2-mercaptoethanol and cultured therein in the presence of 100 µg/ml of keyhole limpet hemocyanin (KLH) for 10 days. Vital cells were collected again by concentration gradient centrifugation with the use of a Ficoll-Urografin solution and introduced into an RPMI-1640 medium containing 10 % of bovine fetal serum with the plastics adherent cells. Then these cells were subcultured therein in the presence of 100 µg/ml of KLH and 5 % of IL-2 while replacing the medium by a fresh RPMI-1640 medium containing 10 % of bovine fetal serum and 5 % of IL-2 at an interval of five days.

(c) Preparation of CEM treated with emetine-actinomycin D

CEM was suspended in an RPMI-1640 medium to give a density of 2 x $10^6$ cells/ml and treated by adding 5 x $10^{-5}$ M of emetine hydrochloride and 0.25 µg/ml actinomycin D at 37°C in the presence of 5 % of $CO_2$ for two hours. After the completion of the treatment, the cells were washed with PBS four times and an MEM medium was added thereto.

(d) Fusion

After subculturing for 30 days, the cells as described in (b) were fused with the CEM treated with emetine-actinomycin D. Thus the subcultured cells of (b) were thoroughly washed to remove KLH, centrifuged and suspended in an MEM medium (pH 7.2) containing 25 mM of HEPES. The CEM treated with emetine-actinomycin D as prepared in (c) was mixed therewith at a ratio of 10 : 1. The mixture was centrifuged to allow to form pellets to which 0.5 ml of an MEM medium containing 46 % of ethylene glycol, 15 % of dimethyl sulfoxide and 5 µg/ml of poly-L-arginine (molecular weight of 60,000) and previously heated to 37°C was added. The mixture was slowly stirred at 37°C for 45 sec and then gradually diluted with an MEM medium containing 25 mM of HEPES to give a volume of 10 ml, The mixture was centrifuged to give pellets which were slowly suspended in a usual medium to give a density of 5 x $10^5$ cells/ml. To 6.0 x $10^6$ of these cells, 1.2 x $10^6$ CEM cells treated with 25 µg/ml of mitomycin C at 37°C for 30 min were added as feeder cells. 0.2 ml portions of this mixture were poured into a plate having 96 wells. 100 µl of the medium in each well was daily replaced by fresh one for one week after the fusion. After several days, the CEM treated with emetine-actinomycin C and those treated with mitomycin C died out while the fused cells in three wells among 20 showed excellent growth two weeks after the fusion.

(e) Cloning

Cloning was carried out by limiting dilution method. That is, a selected KC8 strain was introduced onto a plate having 96 wells to give a density of 0.5 cell/well. At the same time, $2 \times 10^5$/ml of CEM cells treated with 50 µg/ml of mitomycin C for 30 min were added thereto as feeder cells. 10 to 14 days after the addition, the growth of the clone was observed. This strain has been named KC8-1 and ap- plied to deposit with the Fermentation Research Institute.

(f) Determination of surface characters of hybridoma

Surface characters of KC8-1 were determined by two-step binding assay with the use of OKT 3, 4, 8 and 11 (mfd. by Ortho Pharmaceutical Co., Raritan, NJ). As a result it was found that the expression of OKT 8 and 11 was higher when compared with CEM 11 ($p <$ 0.01 in Student's t test). The two-step binding assay is described in detail in reference 9) as cited above.

(g) Purification of lymphokine

KC8-1 was cultured at a density of $10^6$ cells/ml for 20 hours. The supernatant of the culturing broth was collected and saturated to a degree of 90 % with ammonium sulfate. The precipitate thus formed was collected and eluted through a Blue Sepharose column (5.0 ml, mfd. by Pharmacia, Sweden) with PBS. Then the eluate was dialyzed against PBS to give purified lymphokine.

(h) Physicochemical determination

The purified lymphokine obtained in (g) was passed through a TSK G3000-SW column with the use of PBS at a flow rate of 0.25 ml/min. The natural killer cell activity of each fraction was determined.

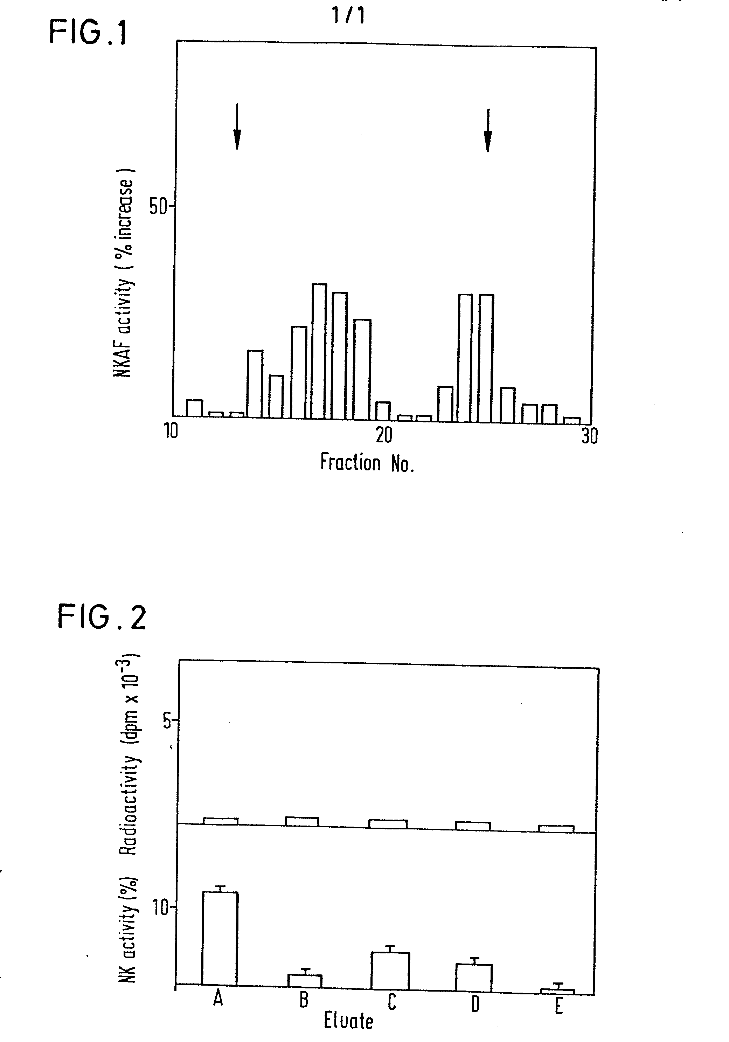

Fig. 1 shows the result wherein the ordinate refers to the NKAF activity calculated according to the equation shown in the process for determining natural killer cell activating factor activity as described above and arrows represent elution positions of standard proteins of known molecular weights, i.e. the left arrow represents the elution position of ovalbumin (44K) while the light arrow represents that of ribonuclease (14K). Fig. 1 suggests that the molecular weights of the lymphokine of the present invention are 15K and 30K.

The natural killer cell activity of the purified lymphokine obtained in (g) was determined immediately after the purification and after allowing to stand at pH 2 for 24 hours. The NK activities thus determined were $33.2 \pm 7.2$ % and $6.1 \pm 5.7$ % respectively while the NK activity determined in the absence of the purified lymphokine was $13.1 \pm 4.2$ %. This result suggests that the lymphokine of the present invention is unstable under an acidic condition.

Similar to the procedure described in (g), a culturing supernatant of KC8-1 was collected and ammonium sulfate was added thereto to give a degree of saturation of 90 %. The precipitate thus formed was collected and eluted stepwise through a Blue Sepharose column with 7.5 ml of PBS (eluent A), 10 ml of PBS (eluent B), 10 ml of a 10 mM phosphate buffer solution (pH 7.0) containing 1.0 M of sodium chloride (eluent C) and a 10 mM phosphate buffer solution (pH 7.0) containing 50 % of ethylene glycol (eluent D). The natural killer cell activity and radioactivity of each fraction were determined by following the procedures as described in the process for determining natural killer cell activating factor activity and in the process for determining IL-2 activity except that the culturing supernatant of human T cell hybridoma was replaced by each fraction eluted with each eluent. Fig. 2 shows the result wherein the upper part of the ordinate refers to the radioactivity while the lower part thereof refers to the natural killer cell activity; and A, B, C and D in the abscissa refer each to a fraction eluted with the corresponding eluent while E refers to a control wherein no fraction is added. Fig. 2 shows that the lymphokine of the present invention exhibits a natural killer cell activating factor activity and no IL-2 activity.

The interferon activity of the purified lymphokine obtained in (g) is not more than 1 IU/ml and can be ignored. Thus the lymphokine of the present invention exhibits no interferon activity.

Example 2

The human T cell hybridoma KC8-1 obtained in Example 1 was cloned by the same limiting dilution method as described in Example 1 - (e) to give seven strains, among which KC8-1-9 and KC8-1-10 have been applied to deposit with the Fermentation Research Institute. The NKAF activities of the culturing supernatants of these two strains were determined. As a result, the NK activity of the former was 9.5 $\pm$ 1.7 % while that of the latter was 9.0 $\pm$ 1.3 % compared with that in the absence of any supernatant (i.e. 6.8 $\pm$ 0.3 %). That is, both exhibit NKAF activities.

In order to confirm that the target of the lymphokine of the present invention is natural killer cells, the

- 22 -

following experiment was carried out.

Experimental Example

Sample

The following plastics nonadherent PBL (A) to (C) were prepared as samples:

(A) plastics nonadherent PBL;

(B) plastics nonadherent PBL treated with antiLeu-11b monoclonal antibody (1 µl/$10^6$ cells) and rabbit complement (mfd. by Cedarlane, Ontario, Canada) and adjusted to the same cell density as that of (A); and

(C) plastics nonadherent PBL treated with rabbit complement (see above) and adjusted to the same cell density as that of (B).

Method

The procedures described in the above process for determining natural killer cell activating factor activity was followed except that the plastics nonadherent PBL was replaced by the sample (A), (B) or (C) and that the culturing supernatant of human T cell hybridoma was replaced by the fraction eluted with the eluent A as shown in Example 1 - (h) to thereby determine the natural killer cell activities S and R. However the final concentration of the lymphokine of the present invention was adjusted to 20 %.

Result

Table 1 shows the result.

Table 1

| Sample | S (%) | | R (%) | |
|--------|-------|------|-------|------|
| (A) | 15.5 | 3.3 | 8.0 | 3.0 |
| (B) | 4.3 | 0.7 | 0.2 | 2.7 |
| (C) | 16.5 | 1.5 | 10.2 | 4.2 |

As shown in Table 1 - (B), the natural killer cell activity is lowered by removing natural killer cells having Leu-11 surface antigen. Thus it is evident that the lymphokine of the present invention requires natural killer cells as the target.

CLAIMS:

- 1 -

A lymphokine which has been produced from human T cell hybridoma derived from human T cells treated with keyhole limpet hemocyanin and has the following physicochemical properties:

(i) it has a molecular weight of approximately 15,000 and 30,000,

(ii) it is chemically unstable under an acidic condition,

(iii) it exerts a natural killer cell activating activity,

(iv) it exhibits no interleukin 2 activity, and

(v) it exhibits no interferon activity.

- 2 - A lymphokine as claimed in Claim 1 in which said human T cells originate from the human peripheral blood.

- 3 -

A lymphokine as claimed in Claim 2 in which said human T cell hybridoma is KC8-1.

- 4 - A lymphokine as claimed in Claim 2 in which said human T cell hybridoma is KC8-1-9 and KC8-1-10.

FIG.1

FIG.2